(19) Europäisches Patentamt European Patent Office Office européen des brevets

(11) **EP 3 941 344 B1**

(12) **EUROPEAN PATENT SPECIFICATION**

(45) Date of publication and mention of the grant of the patent:
**10.06.2026 Bulletin 2026/24**

(21) Application number: **20848515.1**

(22) Date of filing: **29.07.2020**

(51) International Patent Classification (IPC):
**A61B 5/024** (2006.01)   **A61B 5/00** (2006.01)

(52) Cooperative Patent Classification (CPC):
**A61B 5/02416; A61B 5/681; A61B 5/6817; A61B 5/721; A61B 5/7221; A61B 5/7264;** A61B 2562/0219; A61B 2562/046

(86) International application number:
**PCT/US2020/044051**

(87) International publication number:
**WO 2021/021920 (04.02.2021 Gazette 2021/05)**

(54) **METHODS AND APPARATUS FOR DYNAMICALLY IDENTIFYING AND SELECTING THE BEST PHOTOPLETHYSMOGRAPHY SENSOR CHANNEL DURING MONITORING**

VERFAHREN UND VORRICHTUNG ZUR DYNAMISCHEN IDENTIFIZIERUNG UND AUSWAHL DES BESTEN PHOTOPLETHYSMOGRAPHISCHEN SENSORKANALS WÄHREND DER ÜBERWACHUNG

PROCÉDÉS ET APPAREIL PERMETTANT D'IDENTIFIER DE MANIÈRE DYNAMIQUE ET DE SÉLECTIONNER LE MEILLEUR CANAL DE DÉTECTION DE PHOTOPLÉTHYSMOGRAPHIE PENDANT UN CONTRÔLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.07.2019 US 201962880442 P**

(43) Date of publication of application:
**26.01.2022 Bulletin 2022/04**

(73) Proprietor: **Yukka Magic LLC Wilmington DE 19801 (US)**

(72) Inventors:
• **TANK, Tushar Dilip Raleigh, North Carolina 27613 (US)**
• **HODGES, Ryan David Raleigh, North Carolina 27604 (US)**
• **MONCOURTOIS, Nicole Taylor Raleigh, North Carolina 27616 (US)**
• **LEBOEUF, Steven Francis Raleigh, North Carolina 27603 (US)**

(74) Representative: **Bardehle Pagenberg Partnerschaft mbB Patentanwälte Rechtsanwälte Prinzregentenplatz 7 81675 München (DE)**

(56) References cited:
EP-A1- 1 297 784     US-A1- 2002 022 785
US-A1- 2017 071 487     US-A1- 2017 071 487
US-A1- 2017 311 825     US-A1- 2018 353 075
US-A1- 2018 353 075

## Description

This application claims the benefit of and priority to U.S. Provisional Patent Application No. 62/880,442 filed July 30, 2019.

FIELD OF THE INVENTION

The present invention relates generally to monitoring devices and methods, more particularly, to monitoring devices and methods for measuring physiological information.

BACKGROUND OF THE INVENTION

During photoplethysmography (PPG) sensing of a subject via a device worn by the subject, blood flow and motion artifacts are inherently non-uniform across the skin of the subject. As such, the PPG signal from the PPG sensor is also non-uniform across the skin of the subject. Because of this nonuniformity, some regions of the skin are better for PPG monitoring than others, as discussed in U.S. Patent No. 8,251,903.

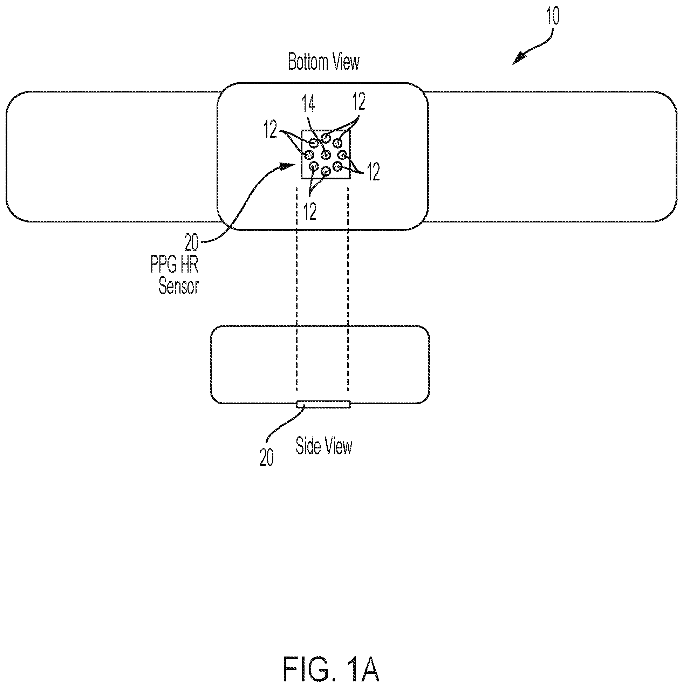

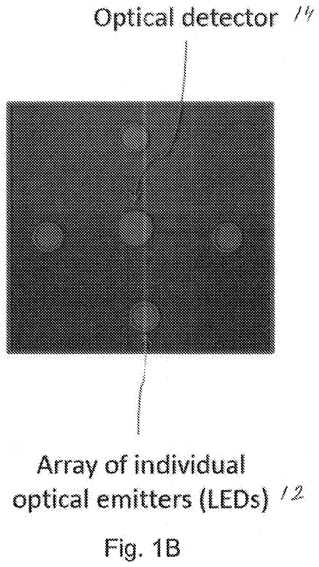

In a wearable PPG monitoring device, such as the arm-worn (or wrist-worn) device 10 shown in Fig. 1A, optical emitter-detector arrays create multiple PPG channels (i.e., optical beam paths through physiological material, such as the skin). Optical emitter-detector arrays can have various numbers of emitters and detectors. For example, the device illustrated in Fig. 1A includes a single detector 14 surrounded by eight emitters 12. Fig. 1B illustrates an emitter-detector array wherein an optical detector 14 is surrounded by four optical emitters 12. Each PPG channel comprises a path starting from an optical emitter 12 and ending at an optical detector 14. Each emitter-detector path, or a combination of paths, can comprise a PPG channel. Thus, for the PPG sensor associated with the four-emitter device of Fig. 1B, there are 15 possible PPG channels for the illustrated emitter-detector configuration.

Each PPG channel can be individually biased, sequentially in time, such that only one PPG channel, ideally the best channel, is "on" at any given time during PPG monitoring. There are several benefits to this methodology. First, selecting the best PPG channel, rather than processing all channels, can reduce electrical power requirements and enhance battery life of a monitoring device. Second, rejecting poor-quality PPG channels can improve the accuracy of a PPG-based biometric measurement, such as heart rate, breathing rate, etc.

However, there are challenges with existing methods used to identify the best PPG channel for PPG monitoring. Typically, the best PPG channel is thought to be the channel having the highest signal-to-noise ratio, and an algorithm is utilized to "hunt" for the PPG channel having the best signal-to-noise ratio at the beginning of a physiological measurement of a subject, identify the channel, and then process only data from this channel throughout the physiological measurement period. However, the signal-to-noise ratio of a PPG signal alone cannot identify the best performing PPG channels of a PPG sensor, and so, often times, a non-optimal channel is selected. Moreover, the signal-to-noise ratio analysis is a static process performed during an initialization period at wearable device startup, and the signal-to-noise ratio analysis is not a dynamic process throughout the entire measurement period of the monitoring device. However, the signal-to-noise ratio of a plurality of PPG sensor channels may change over time. As such, identifying the best PPG channel during an initialization period of a wearable device may not guarantee that a selected PPG channel will remain the optimal PPG channel throughout a measurement period.

SNR techniques are discussed, for instance, in US 2018/353075 (Boston Scientific). A histogram clustering technique for extracting a pulse rate from multiple inputs is disclosed in EP 1297784 (CSEM). Ways of compensating for motion artefacts are discussed in US 2017/071487 (Whoop, Inc.).

SUMMARY

It should be appreciated that this Summary is provided to introduce a selection of concepts in a simplified form, the concepts being further described below in the Detailed Description. This Summary is not intended to identify key features or essential features of this disclosure, nor is it intended to limit the scope of the invention, which is defined in the claims.

Embodiments of the present invention provide dynamic PPG channel identification/selection systems and methods. A plurality of PPG channels of a wearable PPG sensing device can be monitored in real time, via a multivariate probabilistic model, to dynamically identify the best channel for PPG monitoring and to dynamically select the identified best channel for processing biometric parameters. Embodiments of the present invention enable more accurate monitoring for wearable devices, and, in some cases, at a lower power budget than prior solutions.

According to some embodiments of the present invention, a method of identifying a best one of a plurality of photoplethysmography (PPG) channels of a PPG sensor attached to a subject is provided. The PPG sensor includes at least one optical detector and a plurality of optical emitters that define the plurality of PPG channels. The method includes the steps of a) sensing PPG data from the subject using each of the plurality of PPG channels; b) processing the PPG data from each PPG channel, via a processor, to generate a plurality of PPG parameters; and c) processing the PPG parameters, via the processor using a probabilistic model, to identify a best one of the plurality of PPG channels. In some embodiments, the plurality of PPG parameters are selected from the following: a ratio of a magnitude of a pulsatile portion of a PPG waveform to a magnitude of a non-pulsatile portion of the PPG waveform; the non-pulsatile portion of the PPG waveform; a ratio of the magnitude of the pulsatile portion of the PPG waveform to a magnitude of rapid changes in the pulsatile portion of the PPG waveform due to motion artifacts; confidence in quality of a PPG waveform; a ratio of a magnitude of rapid changes in the non-pulsatile portion of the PPG waveform due to motion artifacts to the magnitude of the non-pulsatile portion of the PPG waveform; a frequency at which a peak magnitude of the PPG waveform occurs; a magnitude of a largest spectral peak of the PPG waveform; and a ratio of the peak magnitude of the PPG waveform to a range of spectral magnitudes of the PPG waveform.

The identified best one of the plurality of PPG channels is determined by the probabilistic model to be least likely to generate an error value above a threshold for at least one biometric. The method further includes processing data from the identified best one of the plurality of PPG channels to generate at least one biometric, such as subject heart rate, subject breathing rate, breathing volume, subject RR-interval (RRi), subject blood pressure, subject blood oxygenation, subject hemodynamics, subject blood flow volume, and subject tissue perfusion.

In some embodiments, the PPG sensor further includes at least one motion sensor, and the method further includes sensing motion data via the at least one motion sensor. Processing the PPG data from each PPG channel, via the processor, to generate the plurality of PPG parameters further includes processing the motion data from the at least one motion sensor. In some embodiments, the method of identifying a best one of a plurality of PPG channels of a PPG sensor includes repeating steps a) through c) continuously during a subject monitoring session.

In some embodiments, the method of identifying a best one of a plurality of PPG channels of a PPG sensor further includes determining if the subject has been at rest for a predetermined period of time, and in response to determining that the subject has been at rest for the predetermined period of time, selecting a last identified best PPG channel as the best one of the plurality of PPG channels and terminating the continuous repeating of steps a) through c). In some embodiments, a motion sensor is utilized to determine if the subject has been at rest for a predetermined period of time by processing motion data from the motion sensor and monitoring motion parameters over the predetermined period of time.

According to some embodiments of the present invention, a monitoring device configured to be attached to a subject includes a photoplethysmography (PPG) sensor configured to measure physiological information from the subject, wherein the PPG sensor comprises at least one optical detector and a plurality of optical emitters that define a plurality of PPG channels, and at least one processor. The at least one processor is configured to obtain PPG data from each of the plurality of PPG channels; process the PPG data from each PPG channel to generate a plurality of PPG parameters; and process the PPG parameters using a probabilistic model to identify a best one of the plurality of PPG channels. The identified best one of the plurality of PPG channels is determined by the probabilistic model to be least likely to generate an error value above a threshold for at least one biometric. The at least one processor is further configured to process data from the identified best one of the plurality of PPG channels to generate at least one biometric, such as subject heart rate, subject breathing rate, subject breathing volume, subject RR-interval (RRi), subject blood pressure, subject blood oxygenation, subject hemodynamics, subject blood flow volume, and subject tissue perfusion. In some embodiments, the plurality of PPG parameters are selected from the following: a ratio of a magnitude of a pulsatile portion of a PPG waveform to a magnitude of a non-pulsatile portion of the PPG waveform; the non-pulsatile portion of the PPG waveform; a ratio of the magnitude of the pulsatile portion of the PPG waveform to a magnitude of rapid changes in the pulsatile portion of the PPG waveform due to motion artifacts; confidence in quality of a PPG waveform; a ratio of a magnitude of rapid changes in the non-pulsatile portion of the PPG waveform due to motion artifacts to the magnitude of the non-pulsatile portion of the PPG waveform; a frequency at which a peak magnitude of the PPG waveform occurs; a magnitude of a largest spectral peak of the PPG waveform; and a ratio of the peak magnitude of the PPG waveform to a range of spectral magnitudes of the PPG waveform.

In some embodiments, the monitoring device further includes at least one motion sensor, and the at least one processor is further configured to process motion data from the at least one motion sensor with the PPG data from each PPG channel to generate the plurality of PPG parameters. In some embodiments, the monitoring device is configured to be positioned at or within an ear of the subject. In other embodiments, the monitoring device is configured to be secured to an appendage of the subject.

According to other embodiments of the present invention, a method of identifying a best one of a plurality of photoplethysmography (PPG) channels of a PPG sensor attached to a subject is provided. The PPG sensor includes at least one optical detector and a plurality of optical emitters that define the plurality of PPG channels. The method comprising the steps of a) sensing PPG data from each of the plurality of PPG channels; b) processing the PPG data from each PPG channel, via a processor, to generate a plurality of PPG parameters; and c) processing the PPG parameters, via a probabilistic model, to determine for each of the plurality of PPG channels a respective probability of generating an error value above a threshold for at least one biometric, wherein a respective one of the plurality of PPG channels having the lowest probability is the best one of the plurality of PPG channels. The method further includes processing data from the identified best one of the plurality of PPG channels to generate at least one biometric, such as subject heart rate, subject breathing rate, breathing volume, subject RR-interval (RRi), subject blood pressure, subject blood oxygenation, subject hemodynamics, subject blood flow volume, and subject tissue perfusion.

In some embodiments, the method of identifying a best one of a plurality of PPG channels of a PPG sensor includes repeating steps a) through c) continuously during a subject monitoring session.

In some embodiments, the method of identifying a best one of a plurality of PPG channels of a PPG sensor further includes determining if the subject has been at rest for a predetermined period of time, and in response to determining that the subject has been at rest for the predetermined period of time, selecting a last identified best PPG channel as the best one of the plurality of PPG channels and terminating the continuous repeating of steps a) through c).

In some embodiments, the PPG sensor includes at least one motion sensor, and the method of identifying a best one of a plurality of PPG channels of the PPG sensor further includes sensing motion data via the at least one motion sensor. Processing the PPG data from each PPG channel, via the processor, to generate the plurality of PPG parameters includes processing the motion data from the at least one motion sensor.

BRIEF DESCRIPTION OF THE DRAWINGS

The accompanying drawings, which form a part of the specification, illustrate various embodiments of the present invention. The drawings and description together serve to fully explain embodiments of the present invention.

Fig. 1A illustrates a wrist-worn device having a PPG sensor and in which embodiments of the present invention can be incorporated.
Fig. 1B illustrates an array of optical emitters surrounding an optical detector.
Fig. 2 illustrates a PPG channel hunting mode system that is configured to identify and select the best PPG channel of a PPG sensor during PPG monitoring, according to some embodiments of the present invention.
Fig. 3 illustrates an ear-worn device having a PPG sensor and in which embodiments of the present invention can be incorporated.
Fig. 4 is a table of parameters used in "hunting mode" for accurate heart rate monitoring by a PPG sensor in both an ear-worn device and a wrist-worn device, according to some embodiments of the present invention.
Fig. 5 illustrates a machine learning model for predicting the best PPG channel in real-time, according to some embodiments of the present invention.

Fig. 6 is a table of results of the machine learning model of Fig. 5 in identifying a best PPG channel of a PPG sensor.
Fig. 7 is a flowchart of operations for identifying and selecting the best PPG channel of a PPG sensor during PPG monitoring, according to some embodiments of the present invention.
Fig. 8 is a table of parameters used in "hunting mode" for accurate heart rate monitoring by a PPG sensor in both an ear-worn device and a wrist-worn device, according to some embodiments of the present invention.
Fig. 9 is a time-domain representation of a PPG waveform from a human subject and illustrates various PPG parameters.
Fig. 10A is a spectral-domain representation of a PPG waveform from a human subject without active motion noise removal.
Fig. 10B is a spectral-domain representation of a PPG waveform from a human subject after active motion noise removal.

DETAILED DESCRIPTION

The present invention will now be described more fully hereinafter with reference to the accompanying figures, in which embodiments of the invention are shown. This invention may, however, be embodied in many different forms and should not be construed as limited to the embodiments set forth herein. Like numbers refer to like elements throughout. In the figures, certain components or features may be exaggerated for clarity, and broken lines illustrate optional features or operations unless specified otherwise. In addition, the sequence of operations (or steps) is not limited to the order presented in the figures and/or claims unless specifically indicated otherwise. Features described with respect to one figure or embodiment can be associated with another embodiment or figure although not specifically described or shown as such. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

It will be understood that although the terms first, second, third, etc. may be used herein to describe various elements/operations, these elements/operations should not be limited by these terms. These terms are only used to distinguish one element/operation from another element/operation. Thus a first element/operation in some embodiments could be termed a second element/operation in other embodiments without departing from the teachings of present inventive concepts. The same reference numerals or the same reference designators denote the same or similar elements throughout the specification.

The term "about", as used herein with respect to a value or number, means that the value or number can vary by +/- twenty percent (20%). The term "remote", as used herein, does not necessarily mean that a remote device is a wireless device or that it is a long distance away from a device in communication therewith. Rather, the term "remote" is intended to reference a device or system that is distinct from another device or system or that is not substantially reliant on another device or system for core functionality. For example, a computer wired to a wearable device may be considered a remote device, as the two devices are distinct and/or not substantially reliant on each other for core functionality. Notwithstanding the foregoing, any wireless device (such as a portable device, for example) or system (such as a remote database for example) is considered remote to any other wireless device or system. The terms "respiration rate" and "breathing rate", as used herein, are interchangeable.

The terms "heart rate" and "pulse rate", as used herein, are interchangeable.

The terms "sensor", "sensing element", and "sensor module", as used herein, are interchangeable and refer to a sensor element or group of sensor elements that may be utilized to sense information, such as information (e.g., physiological information, body motion, etc.) from the body of a subject and/or environmental information in a vicinity of the subject. A sensor/sensing element/sensor module may comprise one or more of the following: a detector element, an emitter element, a processing element, optics, mechanical support, supporting circuitry, and the like. Both a single sensor element and a collection of sensor elements may be considered a sensor, a sensing element, or a sensor module. A sensor/sensing element/sensor module may be configured to both sense information and process that information into one or more metrics. The term "monitoring" refers to the act of measuring, quantifying, qualifying, estimating, sensing, calculating, interpolating, extrapolating, inferring, deducing, or any combination of these actions. More generally, "monitoring" refers to a way of getting information via one or more sensing elements. For example, "blood health monitoring" includes monitoring blood gas levels, blood hydration, and metabolite/electrolyte levels, etc.

The term "physiological" refers to matter or energy of or from the body of a creature (e.g., humans, animals, etc.). In embodiments of the present invention, the term "physiological" is intended to be used broadly, covering both physical and psychological matter and energy of or from the body of a creature.

The term "body" refers to the body of a subject (human or animal) that may wear or otherwise be attached to a monitoring device or sensor, according to embodiments of the present invention.

As used herein, the term "processor" broadly refers to a signal processing circuit or computing system, or processing or computing method, which may be localized and/or distributed. For example, a localized signal processing circuit may comprise one or more signal processing circuits or processing methods localized to a general location, such as to an activity monitoring device. Examples of such devices may comprise, but are not limited to, an earpiece, a headpiece, a finger clip, a toe clip, a limb band (such as an arm band or leg band), an ankle band, a wrist band, a nose band, a sensor patch, apparel (clothing) or the like. Examples of a distributed processing circuit include "the cloud," the internet, a remote database, a remote processor computer, a plurality of remote processing circuits or computers in communication with each other, etc., or processing methods distributed among one or more of these elements. The difference between distributed and localized processing circuits is that a distributed processing circuit may include delocalized elements, whereas a localized processing circuit may work independently of a distributed processing system. Microprocessors, microcontrollers, or digital signal processing circuits represent a few non-limiting examples of signal processing circuits that may be found in a localized and/or distributed system.

The term "health", as used herein, is broadly construed to relate to the physiological status of an organism or of a physiological element or process of an organism. For example, cardiovascular health may refer to the overall condition of the cardiovascular system, and a cardiovascular health assessment may refer to an estimate of blood pressure, VO$_2$max, cardiac efficiency, heart rate recovery, arterial blockage, arrhythmia, atrial fibrillation, or the like. A "fitness" assessment is a subset of a health assessment, where the fitness assessment refers to how one's health affects one's performance at an activity. For example, a VO$_2$max test can be used to provide a health assessment of one's mortality or a fitness assessment of one's ability to utilize oxygen during an exercise.

The term "blood pressure", as used herein, refers to a measurement or estimate of the pressure associated with blood flow of a person.

The term "metric" generally refers to a measurement or measurement system of a property, and a "sensor metric" refers to a measurement or measurement system associated with a sensor. The metric may comprise an identifier for a type of measurement, a value of the measurement, and/or a diagnosis based on the measurement. For example, a metric may comprise "blood pressure", with a value of "120/80", and/or a diagnosis of "normal".

The terms "optical source" and "optical emitter", as used herein, are interchangeable.

The term "coupling", as used herein, refers to the interaction or communication between excitation light entering a region of a body and the region itself. For example, one form of optical coupling may be the interaction between excitation light generated from an optical sensor module and the blood vessels of the body of a user. In one embodiment, this interaction may involve excitation light entering the ear region and scattering from a blood vessel in the ear such that the intensity of scattered light is proportional to blood flow within the blood vessel.

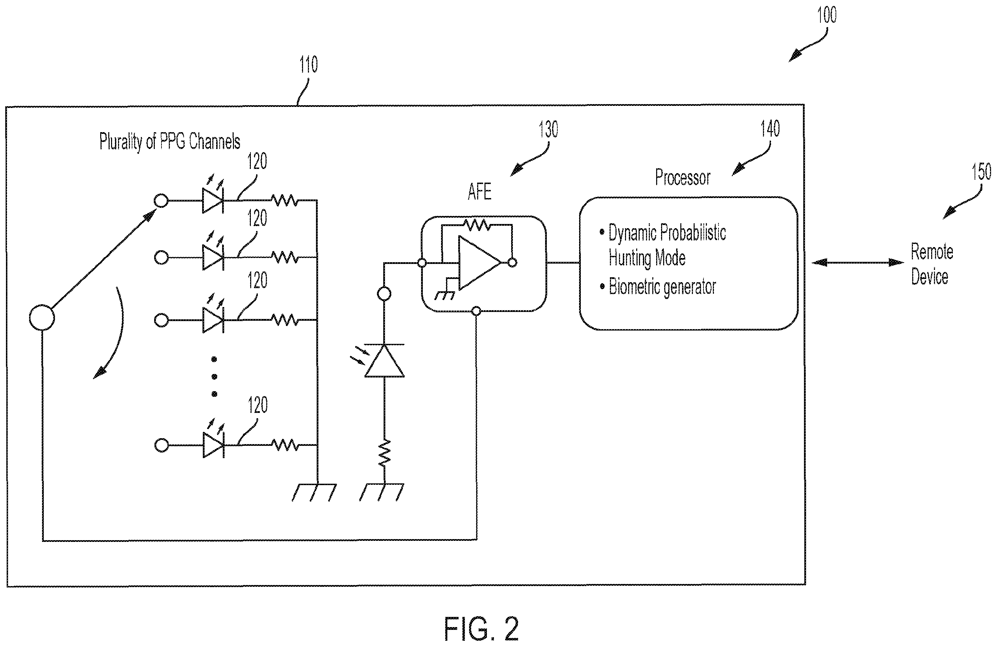

Fig. 2 illustrates a dynamic PPG channel hunting mode system 100, according to some embodiments of the present invention, that is configured to monitor a plurality of PPG channels 120 of a PPG sensor, via a multivariate probabilistic model, and dynamically identify the best channel for PPG monitoring and select the identified best channel for processing biometric parameters. The illustrated PPG channel hunting mode system 100 includes a wearable device 110 having a PPG sensor with a plurality of PPG channels 120 configured to be selectively biased on/off alternating in time, an analog front end (AFE) mechanism 130 for amplifying and digitizing the PPG signals from each PPG channel 120, and a processing mechanism 140 for processing the resulting digitized PPG signals dynamically, in real-time, via a multivariate probabilistic model, to dynamically identify the best channel for PPG monitoring. The processing mechanism 140 is configured to dynamically select the identified best channel for processing biometrics, such as heart rate, RR-interval (RRi), blood pressure, blood oxygenation, hemodynamics, blood flow volume, tissue perfusion, blood analyte level - such as glucose, and the like. The wearable device 110 may communicate with a remote device 150, such as a portable telecommunication device. Though only one optical detector is shown feeding the AFE 130 in Fig. 2, this symbolism is meant to represent either one optical detector or a plurality of optical detectors feeding the AFE 130. Alternatively, at least two optical detectors may feed different AFEs. In principle, any number or combination of emitters, detectors, and AFEs may be used in this invention, as long as a plurality of PPG channels are made available.

The hunting mode system alternately activates a plurality of PPG channels (i.e., modulates each PPG channel of a PPG sensor in time such that only one channel is turned on, or is activated at a certain level, at a given time) to generate a plurality of PPG signals, one from each channel. It should be noted that the term "channel" can refer to one PPG channel or a plurality of PPG channels activated together. A PPG channel is defined by at least one optical pathway between at least on emitter and at least one detector. As such, in some embodiments, all PPG channels are turned on all the time, and only one channel is activated higher than the others at a given time. In other embodiments, multiple channels can be activated at the same time.

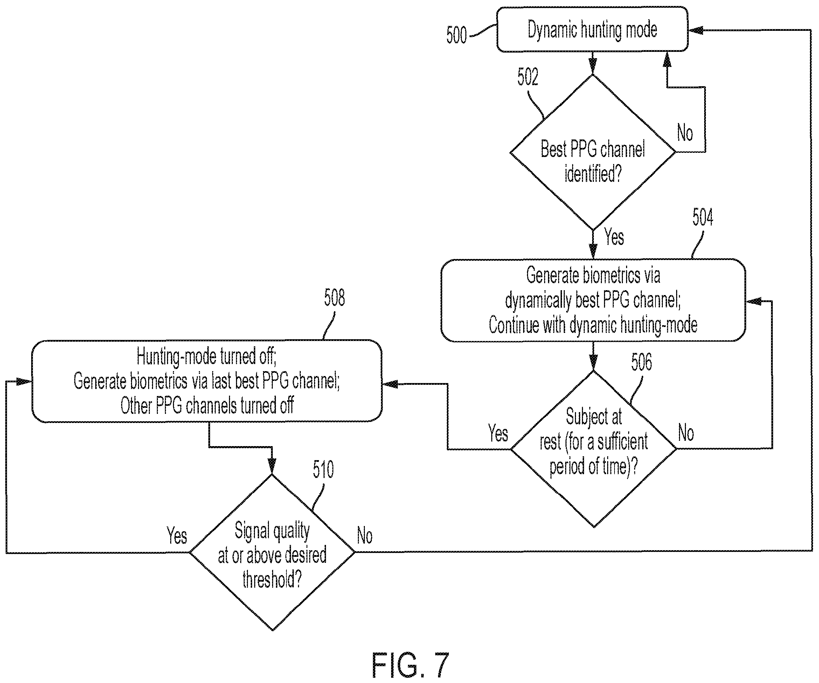

The multivariate probabilistic model of the hunting mode system then processes the PPG signals from each PPG channel dynamically, in real time, to determine which PPG channel has the lowest probability of generating an error value above a threshold for at least one biometric parameter, thereby identifying the most desirable PPG channel as the PPG channel having the lowest said probability. The hunting mode system then selectively processes the most desirable PPG channel to generate and report the at least one biometric parameter. The above steps are then repeated dynamically, in real-time, during monitoring of a subject. In some embodiments, there may be feedback sent to the AFE 130 of Fig. 2, to activate or deactivate (such as to turn on or turn off) a channel deemed desirable or undesirable, as a means of saving power and improving performance (accuracy) in a biometric measurement (such as for the example shown in Fig. 7).

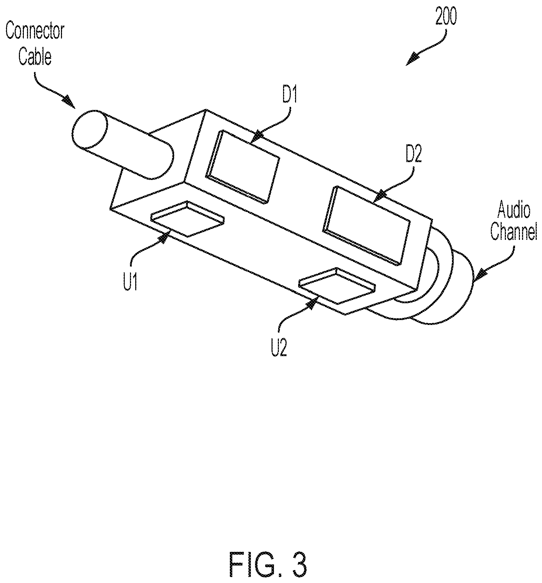

A PPG-enabled RIC (receiver-in-canal) audio driver 200 for hearing aids is illustrated in Fig. 3. The illustrated audio driver 200 (also referred to as an earpiece) includes optical emitters D1, D2 and optical detectors U1, U2 integrated into the device. As such, there are 6 possible PPG channels in this particular configuration, each having a unique optical path: D1 to U1, D1+D2 to U1, D2 to U2, D1+D2 to U2, D1 to U2, and D2 to U1. However, other optical emitters on the other side of the RIC audio driver 200 may also be used to add a plurality of additional PPG channels. Other exemplary configurations for a PPG-enabled audio driver are described in U.S. Patent No. 10,015,582 and U.S. Provisional Patent Application No. 62/733,327.

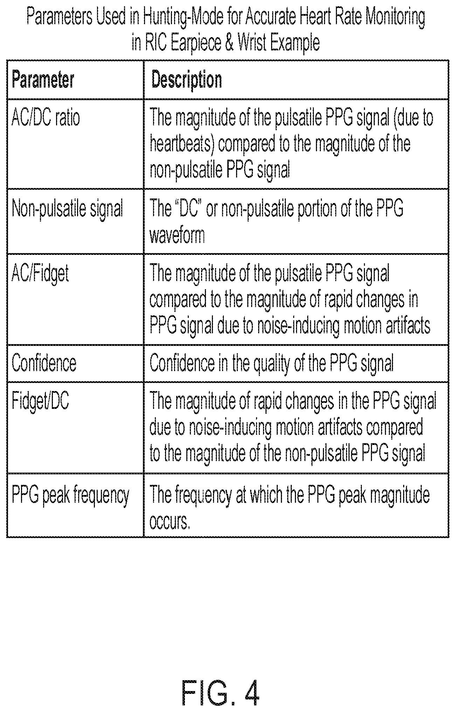

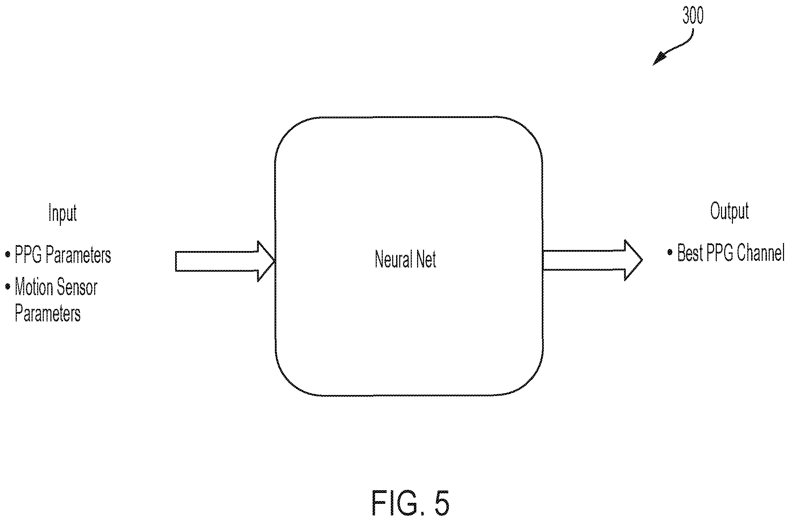

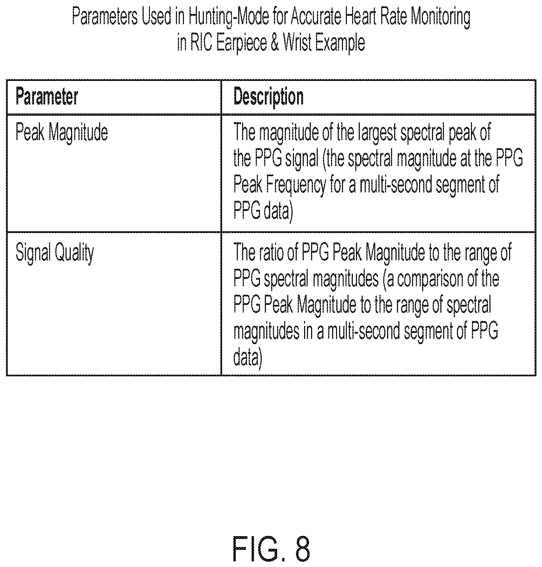

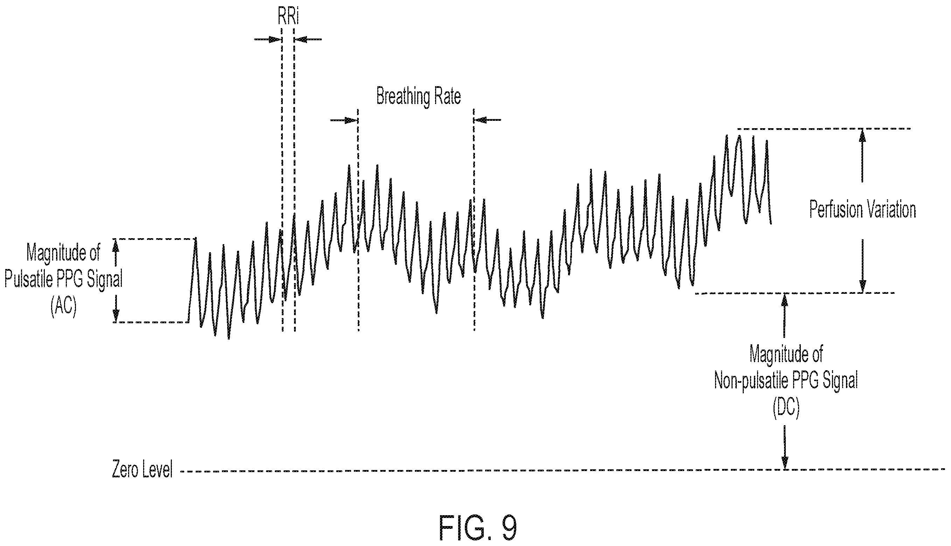

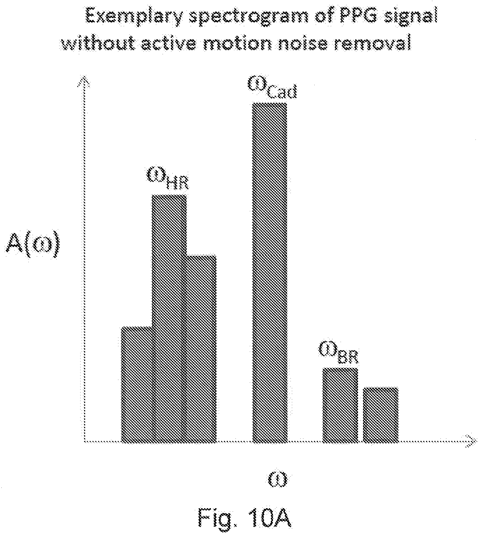

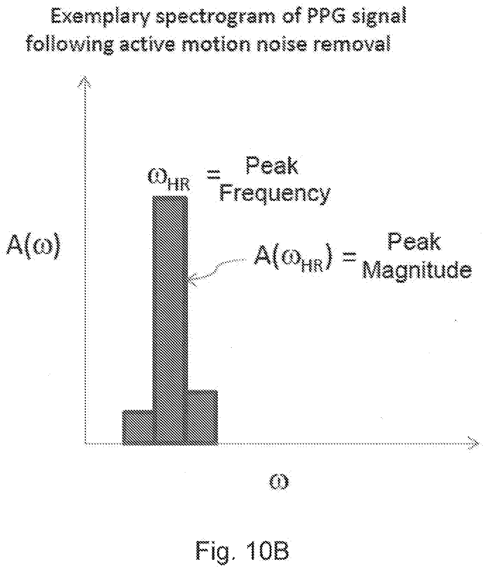

To develop the probabilistic model for predicting the best PPG channel in real-time, a machine learning model 300 was developed that uses inputs comprising various PPG parameters, as summarized in Fig. 4 and in Fig. 8, and illustrated in Figs. 9, 10A and 10B. The machine learning model 300 configuration is presented in Fig. 5. Both PPG data from the earpiece 200 and respective benchmark ECG data from several subjects are entered into the machine learning model 300. For each of the plurality of PPG channels, the neural net of the machine learning model 300 was designed to determine (i.e., to classify) the probability that the PPG channel would yield an error in heart rate (HR) calculation greater than or equal to a certain threshold (i.e., a PPG heart rate error $\geq$ 10 BPM (beats per minute) when compared with ECG data.) In this particular case, the estimation of probabilities was assessed each second, and at each second the PPG channel having the lowest said probability was selected as the best PPG channel for the output of the machine learning model.

It should be noted that the probabilistic model of this invention may comprise a machine learning model, but other types of probabilistic model structures that are not considered machine learning may be used in this invention. The unifying theme of any chosen model structure is that it should be structured to provide information about which of the PPG channels has the greatest or least probability for achieving the desired output.

The term "error" is the difference between the HR estimation (via PPG) and the HR measurement from the ECG chest strap. Similarly, using this same approach to estimate the error for BR (breathing rate), the BR error would be the difference between the BR estimation (via PPG) and the BR measurement from a benchmark BR monitor, such as an impedance sensor, a gas exchange analysis sensor, a video analysis platform, or the like.

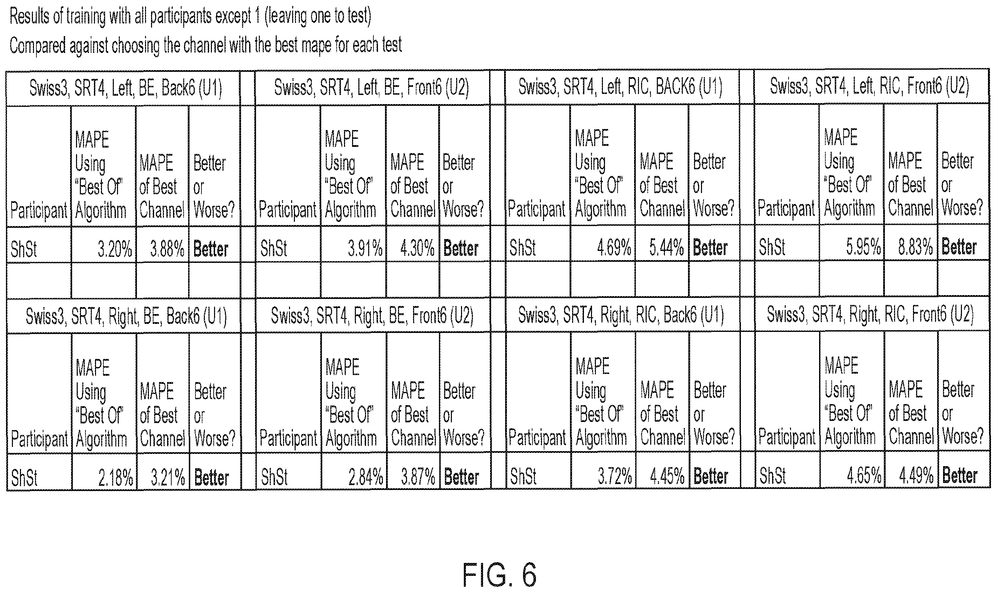

An example of model performance is presented in Fig. 6, where "U1" and "U2" refers to the back and front optical detectors U1, U2 shown in Fig 3. The "right" and "left" nomenclature refers to a left earpiece and a right earpiece worn by a subject. The subjects in this experiment were wearing two of the devices 200 shown in Fig. 3, one device 200 in each ear. The results of Fig. 6 show that the dynamic hunting mode algorithm is superior to simply picking the best PPG channel at the beginning of a session (a static hunting mode). Namely, the MAPE (mean absolute percent error) of the heart rate estimation using the "best of" algorithm (dynamic hunting mode) was notably lower for all participants shown in comparison that of the "best channel" (static hunting-mode).

The machine learning model 300 configuration of Fig. 5 was employed in a second experiment, with an updated set of input parameters and an updated neural net, but using the same PPG datasets as used for Fig. 6.

The updated set of input parameters comprised all of those listed in Fig. 4, but additionally comprised information about the PPG peak magnitude and the signal quality (see Fig. 8), as well as information about the ratio of PPG peak magnitude to that of one or more related harmonics. Before being implemented in the PPG sensor prototype, the updated neural net was retrained based on these additional inputs, with the target of predicting, for each PPG channel, the probability that the heart rate estimation error would be greater than 5 BPM (as opposed to 10 BPM as in the first experiment). As employed in the PPG sensor prototype, the real-time output of the trained neural net was further processed to determine the PPG channel having the lowest said probability. In this particular case, the estimation of probabilities was assessed each second, and the lowest probability was ascribed to the PPG channel having the lowest (minimum) weighted average of these probabilities over 30 seconds. (Namely, a 30 second scrolling buffer was applied to store each 1-sec probability determination). This PPG channel was then deemed to be the best PPG channel for the output of the machine learning model of Fig. 5. The resulting accuracy improvement was substantial: the accuracy of the HR estimation improved from a MAPE of 11.5% (using all 3 emitters at all times) to a MAPE of 6.7% (using the optimal channel). Thus, both performance (accuracy) enhancement and power savings were realized. It was also found that the neural net did not need to be continuously employed, as the best channel would remain "best" for several seconds following determination. For example, for this particular ear-worn PPG sensor prototype, the best channel could be maintained for several seconds (often 15 seconds or more) before the neural net would need to be employed to update the best PPG channel, primarily due to motion artifacts.

Embodiments of the present invention have also been demonstrated in the laboratory in a wrist-worn device. In this device, there were three optical emitters surrounding a central photodiode. The machine learning model for this configuration was generated using 124 datasets from 39 subjects, and inputs to the model included the PPG parameters listed in Fig. 4 and benchmark ECG heart rate values. The algorithm developed from the model was then implemented in the machine learning model 300 of Fig. 5. The results showed that picking the best PPG channel was overall superior to using all of the channels combined, yielding better performance, regardless of which probability threshold (5 or 10 BPM) was implemented.

It should be noted that, for Fig. 5, the input to the machine learning model 300 may comprise PPG parameters only, motion parameters only, or a combination of both. Suitable PPG sensors may comprise a variety of optical emitters (LEDs, LDs, VCSELs, OLEDs, nanoscale emitters, microplasmas, and the like) and optical detectors (photodiodes, photoconductors, phototransistors, cameras, imaging systems, color sensors, and the like). Moreover, suitable motion sensors that may be utilized with PPG sensors include a variety of sensors configured to measure mechanical changes, such as accelerometers, gyroscopes, vibration sensors, a variety of MEMS sensors, motion-sensing cameras, acoustic sensors, optical motion sensors, and the like. However, other PPG sensors and motion sensors may be used. Moreover, other inputs may be used to help improve PPG channel selection accuracy, such as static user biometrics (i.e., height, weight, age, gender, medication usage, and the like), inputs from other sensors, or the like.

Embodiments of the present invention may be implemented as software (i.e., embedded firmware, a library, host software, distributed software, remote software, or the like) within a PPG sensor having multiple PPG channels. The software would collect sensor data and output the best PPG channel for further signal processing. The calculation of "best PPG channel" may be made before or after the processing of the desired biometrics. The benefit of determining the best PPG channel via the inventive aspects of Fig. 2 and Fig. 5 without first processing a heart rate (or another biometric) is that calculating heart rate for each PPG channel can be costly for battery life. Preferably, a determination of the best PPG channel could be made without calculating heart rate for each channel, such that only the best channel would require heart rate signal processing. In such case, the selected PPG parameters should be lightly processed parameters, that require relatively light battery power in comparison to heart rate determination.

Examples of low-power-requiring parameters may include changes in DC PPG magnitude, autocorrelation values, and the like. Once the best PPG channel is selected, then heart rate processing (or processing of another biometric) may be implanted for only that channel. Alternatively, the neural net of Fig. 5 may be a convolutional neural net operating on raw PPG data to generate an assessment of the best PPG channel. Although this method may lead to a very low power dynamic hunting-mode solution, a weakness of this approach may be that optimal system performance may require new machine learning training for each new PPG sensor configuration used. This may limit the scalability of the dynamic hunting-mode solution for a plurality of wearable devices in the mass market. Thus, trade-offs must be considered, depending on the use case implementation.

The machine learning model 300 may also be built using parameters as presented in Fig. 4, where these parameters are expected to be calculated nearly the same way for each PPG channel and device configuration.

This was the approach used in the first and second experiments summarized above. A key benefit of this approach is that the machine learning probabilistic model may be considerably more scalable, as the PPG parameters may be the same for any given sensor configuration (on the same subject and using the form-factor). This may enable the PPG sensor hardware to be interchangeable with a variety of wearable products, making a scalable software solution viable.

However, in some cases this approach may be more power consuming, depending on the use case implementation.

The parameters of Fig. 4 and Fig. 8 are provided as non-limiting examples, and a wide variety of parameters may be used to create the probabilistic model for dynamic hunting-mode. One non-limiting class of suitable parameters may include transforms of the sensor data, such as spectral transforms, wavelet transforms, derivatives, integrals, and the like. Additionally, the waveforms collected from the sensor itself may be used as a parameter. In such case, as with transforms of the waveform, using the waveform as a parameter itself may comprise generating a representation of the waveform that can be processed. For example, the waveform data (or a transform of the waveform data) from the collected sensor data may be represented as a set number of values at a set number of points in time rather than a "continuous" set of data in time. Thus, the parameters used by the probabilistic model to execute dynamic hunting-mode may comprise these set values for these set points in time. It should also be noted that motion parameters may also be used as inputs to the probabilistic model, such as (but not limited to) parameters of subject cadence (step rate), speed, pace, distance, accelerometer waveforms, accelerometer waveform transforms, accelerometer counts, an integral of accelerometer counts, average accelerometer readings over a period of time, accelerometer RMSD (root-mean-squared deviation) readings, and the like.

It should be noted that the PPG parameters of Fig. 4 and Fig. 8 can be utilized with and without motion noise removal. PPG motion noise removal may be executed via a variety of different methods well known to those skilled in the art. In the case of the first and second experimental results described above, the PPG parameters were utilized after motion noise removal, such that the PPG peak magnitude comprised the peak PPG spectral magnitude following the spectral subtraction of motion noise (as sensed by the motion sensor), using a method similar to that presented in U.S. Patent No. 10,631,740 and U.S. Patent No. 8,888,701.

The PPG parameters of Figs. 4 and 8 are presented graphically in Figs. 9, 10A and 10B. Fig. 9 illustrates a time-domain representation of a PPG waveform from a human subject, and Figs. 10A-10B present a spectral-domain representation of a PPG waveform from a human subject. Note from Fig. 9 that the "magnitude of the pulsatile PPG signal" (the PPG "AC" signal) is presented as the time-domain magnitude of the PPG heartbeat pulse. These magnitudes may be used individually or they may be averaged over a period of time (i.e., over a buffering period) to be utilized as a parameter in this invention.

Alternatively, a spectral representation may be used, as shown in Figs. 10A-10B, where the magnitude of the pulsatile signal is represented as the spectral magnitude of the of the heart rate signal - the peak magnitude $A(\omega_{HR})$.

Following motion cancellation, the peak magnitude may typically be the same as the spectral magnitude of the HR component of the PPG waveform. This "peak magnitude" may be associated with a "PPG peak frequency" $\omega_{HR}$, the frequency of the subject's heartbeat. In contrast, the non-pulsatile signal (the PPG "DC" signal) does not have a spectral analogue, and therefore it may be addressed by calculating the DC offset (the "magnitude of the non-pulsatile PPG signal") in the time-domain, as shown in Fig. 9. Thus, the AC/DC ratio may comprise a ratio of the spectral magnitude of the pulsatile PPG signal to the time-domain magnitude of the non-pulsatile signal, or alternatively it may comprise a ratio of the time-domain magnitude of the pulsatile PPG signal to the time-domain magnitude of the non-pulsatile PPG signal. It should be noted that, more broadly, a variety of representations of AC/DC (pulsatile/non-pulsatile) ratio may be used to feed the hunting mode model. An important common element is that the method of representing this ratio should be consistent for all datasets both during training of the model and during algorithm execution in a real-world PPG device.

The "fidget" parameter illustrated in Fig. 4 may be generated through a variety of means, with the goal of capturing the magnitude of rapid changes in the PPG signal due to noise-inducing motion artifacts. As noted above, an important common element is that the method of generating "fidget" information should be consistent for all datasets during training and in the field (real-world environment). For the results summarized herein, the fidget information was generated by averaging the absolute values of the magnitudes of oscillations in the PPG signal. These oscillations may be solely due to biometric changes (i.e., changes in heart rate, breathing rate, blood pressure, and the like) during resting periods for the subject. But during motion, these oscillations may be dominated by motion artifacts.

The "confidence" and "signal quality" parameters illustrated in Fig. 4 and Fig. 8 may be generated through a variety of methods, and a few examples are presented in U.S. Patent No. 9,794,653. For the results summarized herein, the confidence and signal quality were calculated using methods presented in U.S. Patent No. 9,794,653. An exemplary formula for signal quality presented in Fig. 8 is the following:

$$\text{Signal Quality} = Q_S \; \alpha \; \frac{(A(\omega_{HR}))}{\sum A(\omega_i)}$$

In other words, signal quality Qs is proportional to the spectral magnitude of the heart rate signal divided by the sum of the signal components (the sum of all spectral amplitudes for all of the "n" discrete frequencies $\omega_i$, for i = 0 to n), some of which may be associated with user motion. This formula may be useful once a spectrogram is generated for a PPG signal. In the spectral domain, the signal quality Qs may be expressed as a ratio of the spectral amplitude at the HR (heart rate) frequency divided by a sum of various other spectral amplitudes that may also exist in the spectrogram. Similarly, the signal quality Qs may be related to a ratio of functions of various spectral amplitudes. The signal quality Qs may be assessed either before or after motion-artifact removal, but in the case of assessing signal quality post-motion-artifact removal, the sum of spectral amplitudes in the denominator is likely to be smaller than for the case of assessing signal quality pre-motion-artifact removal. Thus, when there are less spectral artifacts from motion artifacts and other unwanted time-varying artifacts, the signal quality Qs is likely to be higher than for the case where many such artifacts are present.

The above formula for assessing signal quality is not meant to be limiting. Various other formulas or methods may be used to assess signal quality according to embodiments of the present invention. As just one example, a time-domain or wavelet representation for signal quality (using a ratio as shown above) may be used in place of a spectral representation, with the numerator comprising amplitude information about the heart rate component of the signal and the dominator comprising magnitude information about all signals. Other signal quality formalisms may be used, and signal quality does not necessarily need to compare signal magnitudes to be useful for the probabilistic model.

It should also be noted that the choice of signal quality formula may depend on the biometric parameter of most relevance to the probabilistic model. For example, if the probabilistic model is configured to pick out the best PPG channel for breathing rate (BR) as opposed to heart rate, the formula above should utilize $A(\omega_{BR})$ rather than $A(\omega_{HR})$ in the numerator. Alternatively, both signal quality inputs may be incorporated into the probabilistic model, to help identify the best PPG channels for both heart rate and breathing rate. Other signal quality inputs from other biometric parameters may also be used.

Wearable devices in which a PPG sensor may be incorporated in accordance with embodiments of the present invention include earbuds, headphones, headsets, hearing aids, other earpieces, headbands, armbands, wristbands, eyewear, leg bands, neckbands, body jewelry, tattoos, finger or toe rings, patches, apparel/clothing, and the like. Other form-factors may also be used.

In some embodiments, to improve accuracy while also reducing battery power, hunting mode may be implemented to identify the best PPG channel, and then the hunting mode functionality and the undesired PPG channels may be deactivated (i.e., turned off), once it is determined that the subject has been at rest for a sufficient period of time. This determination can be made by processing motion sensor data to determine a user's activity state. The rationale for this approach is that it is unlikely that the best PPG channel will change during periods of rest, as motion artifacts will not be present, and thus there may be no real need to continually process hunting mode and unnecessary PPG channels during periods of rest. When physical activity resumes, the motion sensor information can be used to determine that subject is no longer at rest for a sufficient period of time (phrased another way, the processing of motion sensor readings may show that the subject has moved or is moving for a significant duration). In such case, the hunting mode procedure may be restarted to identify the best PPG channel (dynamically, throughout the entire physical activity period). Thus, the dynamic hunting mode is activated during subject activity and deactivated during subject rest, as shown in Fig. 7.

Fig. 7 illustrates operations for identifying and selecting the best PPG channel of a PPG sensor during PPG monitoring, according to some embodiments of the present invention. Dynamic hunting mode begins at Block 500, and a determination is made whether the best PPG channel has been identified (Block 502). If the answer is no, dynamic hunting mode (Block 500) continues until the best PPG channel is identified. When the best PPG channel is identified, biometrics are generated via the identified best PPG channel (Block 504). Dynamic hunting mode also continues (Block 504).

A determination is then made whether the subject is at rest for a period of time (Block 506). If the answer is yes, hunting mode is turned off and biometrics are generated via the last identified best PPG channel while the other PPG channels are turned off (Block 508). If signal quality is acceptable (Block 510), hunting mode remains off and biometrics are generated via the last identified best PPG channel while other PPG channels remain turned off (Block 508). If signal quality is not acceptable, then dynamic hunting mode is turned back on (Block 500). It should be noted that signal quality may be assessed in various ways. For example, the output of an accelerometer (or other motion sensor) may be monitored to see if the average motion for the subject is below a threshold. If so, then the signal quality may be acceptable. As another example, the signal quality of the PPG signal may be monitored by processing the PPG and motion signals to determine the ratio of pulsatile signal ("AC") to baseline signal ("DC"), wherein a higher signal quality is associated with a higher AC/DC ratio, and a ratio above a threshold signifies an acceptable signal quality. A variety of signal quality analysis methods are known to those skilled in the art, and a few examples are provided in U.S. Patent No. 9,794,653.

It is unlikely that the best PPG channel will change under subject resting conditions; but, during motion, the optomechanical coupling between the skin of the user and at least one PPG channel may change. Thus, by turning off all non-optimal PPG channels during rest, a substantial amount of power may be saved, and indeed, the power budget may improve substantially over a PPG wearable having all PPG channels turned on continuously. It should be understood that the time periods between activation/deactivation during subject activity/rest transitions need not be instantaneous, and a suitable transition time of a few seconds between activity and resting transitions may be sufficient.

It should be noted that a variety of analog and/or digital processing methodologies may be employed in the invention herein, including, but not limited to, classical digital signal processing, machine learning methodologies, biologically inspired circuits, analog switching networks, neural circuits, and the like. The processing may be linear or nonlinear. In a preferred model, the probabilistic model for dynamic hunting mode may be created via a machine learning approach (i.e., neural networks, random forests, convolutional neural nets, binary trees, and the like), as described with reference to Fig. 4, Fig. 5, and Fig. 6.

Example embodiments are described herein with reference to block diagrams and flowchart illustrations. It is understood that a block of the block diagrams and flowchart illustrations, and combinations of blocks in the block diagrams and flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor circuit and/or other programmable data processing apparatus, transform and control transistors, values stored in memory locations, and other hardware components within such circuitry to implement the functions/acts specified in the block diagrams and flowchart block or blocks, and thereby create means (functionality) and/or structure for implementing the functions/acts specified in the block diagrams and flowchart blocks.

These computer program instructions may also be stored in a tangible computer-readable medium that can direct a client device or other programmable data processing apparatus to function in a particular manner, such that the instructions stored in the computer-readable medium produce an article of manufacture including instructions which implement the functions/acts specified in the block diagrams and flowchart blocks.

A tangible, non-transitory computer-readable medium may include an electronic, magnetic, optical, electromagnetic, or semiconductor data storage system, apparatus, or device. More specific examples of the computer-readable medium would include the following: a portable computer diskette, a random access memory (RAM) circuit, a read-only memory (ROM) circuit, an erasable programmable read-only memory (EPROM or Flash memory) circuit, a portable compact disc read-only memory (CD-ROM), and a portable digital video disc read-only memory (DVD/BlueRay).

The computer program instructions may also be loaded onto a client device and/or other programmable data processing apparatus to cause a series of operational steps to be performed on the client device and/or other programmable apparatus to produce a computer-implemented process such that the instructions which execute on the client device or other programmable apparatus provide steps for implementing the functions/acts specified in the block diagrams and flowchart blocks. Accordingly, embodiments of the present invention may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and block diagrams may be separated into multiple blocks and/or the functionality of two or more blocks of the flowcharts and block diagrams may be at least partially integrated. Finally, other blocks may be added/inserted between the blocks that are illustrated. Moreover, although some of the diagrams include arrows on communication paths to show a primary direction of communication, it is to be understood that communication may occur in the opposite direction to the depicted arrows.

The foregoing is illustrative of the present invention and is not to be construed as limiting thereof. Although a few exemplary embodiments of this invention have been described, those skilled in the art will readily appreciate that many modifications are possible in the exemplary embodiments without materially departing from the teachings and advantages of this invention.

**Claims**

1. A method of identifying a best one of a plurality of photoplethysmography, PPG, channels of a PPG sensor attached to a subject, wherein the PPG sensor comprises at least one optical detector (14) and a plurality of optical emitters (12) that define the plurality of PPG channels (120), the method comprising the steps of:

   a) sensing PPG data from the subject using each of the plurality of PPG channels for a time period; and
   b) processing the PPG data from each PPG channel, via a processor, to generate a plurality of PPG parameters;

   **characterized by** the further step:

   c) processing the PPG parameters, via the processor using a probabilistic model, to identify for the time period, a PPG channel of the plurality of PPG channels having a lowest probability of generating an error value above a threshold for at least one biometric, wherein the probabilistic model comprises a machine learning model configured to receive the PPG parameters as input and output a probability that a PPG channel is associated with an error rate greater than a defined threshold.

2. The method of Claim 1, wherein the method further comprises processing data from the identified best one of the plurality of PPG channels to generate at least one biometric;
   wherein optionally the at least one biometric further comprises at least one of subject breathing rate, breathing volume, subject RR-interval (RRi), subject blood pressure, subject blood oxygenation, subject hemodynamics, subject blood flow volume, and subject tissue perfusion.

3. The method of Claim 1, wherein the PPG sensor further comprises at least one motion sensor, and wherein the method further comprises:

   sensing motion data via the at least one motion sensor; and
   wherein processing the PPG data from each PPG channel, via the processor, to generate the plurality of PPG parameters comprises processing the motion data from the at least one motion sensor.

4. The method of Claim 1, wherein each PPG parameter comprises PPG waveform data or a transform of PPG waveform data.

5. The method of Claim 1, further comprising the step of:
   d) repeating steps a) through c) continuously during a subject monitoring session.

6. The method of Claim 5, further comprising determining if the subject has been at rest for a predetermined period of time, and in response to determining that the subject has been at rest for the predetermined period of time, selecting a last identified PPG channel as the identified PPG channel of the plurality of PPG channels and terminating step d);
   and optionally wherein the monitoring device comprises at least one motion sensor, and wherein determining if the subject has been at rest for a predetermined period of time comprises processing motion data from the at least one motion sensor and monitoring motion parameters over the predetermined period of time.

**7.** The method of Claim 1, wherein the plurality of PPG parameters are selected from the following:

a ratio of a magnitude of a pulsatile portion of a PPG waveform to a magnitude of a non-pulsatile portion of the PPG waveform;
the non-pulsatile portion of the PPG waveform;
a ratio of the magnitude of the pulsatile portion of the PPG waveform to a magnitude of rapid changes in the pulsatile portion of the PPG waveform due to motion artifacts;
confidence in quality of a PPG waveform;
a ratio of a magnitude of rapid changes in the non-pulsatile portion of the PPG waveform due to motion artifacts to the magnitude of the non-pulsatile portion of the PPG waveform;
a frequency at which a peak magnitude of the PPG waveform occurs;
a magnitude of a largest spectral peak of the PPG waveform; and
a ratio of the peak magnitude of the PPG waveform to a range of spectral magnitudes of the PPG waveform.

**8.** A monitoring device configured to be attached to a subject, the monitoring device comprising:

a photoplethysmography, PPG, sensor configured to measure physiological information from the subject, wherein the PPG sensor comprises at least one optical detector (14) and a plurality of optical emitters (12) that define a plurality of PPG channels (120); and
at least one processor configured to:

obtain PPG data for a time period from each of the plurality of PPG channels; and
process the PPG data from each PPG channel to generate a plurality of PPG parameters for the time period;

**characterized in that** the processor is further configured to:
process the PPG parameters using a probabilistic model to identify for the time period a PPG channel of the plurality of PPG channels having a lowest probability of generating an error value above a threshold for at least one biometric, wherein the probabilistic model comprises a machine learning model configured to receive the PPG parameters as input and output a probability that a PPG channel is associated with an error rate greater than a defined threshold.

**9.** The monitoring device of Claim 8, wherein the at least one processor is further configured to process data from the identified PPG channel of the plurality of PPG channels to generate at least one biometric, wherein the at least one biometric comprises at least one of subject heart rate, subject breathing rate, subject breathing volume, subject RR-interval, RRi, subject blood pressure, subject blood oxygenation, subject hemodynamics, subject blood flow volume, and subject tissue perfusion.

**10.** The monitoring device of Claim 8, further comprising at least one motion sensor, and wherein the at least one processor is further configured to process motion data from the at least one motion sensor with the PPG data from each PPG channel to generate the plurality of PPG parameters.

**11.** The monitoring device of Claim 8, wherein each PPG parameter comprises PPG waveform data or a transform of PPG waveform data.

**12.** The monitoring device of Claim 8, wherein the plurality of PPG parameters are selected from the following:

a ratio of a magnitude of a pulsatile portion of a PPG waveform to a magnitude of a non-pulsatile portion of the PPG waveform;
the non-pulsatile portion of the PPG waveform;
a ratio of the magnitude of the pulsatile portion of the PPG waveform to a magnitude of rapid changes in the pulsatile portion of the PPG waveform due to motion artifacts;
confidence in quality of a PPG waveform;
a ratio of a magnitude of rapid changes in the non-pulsatile Portion of the PPG waveform due to motion artifacts to the magnitude of the non-pulsatile portion of the PPG waveform;
a frequency at which a peak magnitude of the PPG waveform occurs;
a magnitude of a largest spectral peak of the PPG waveform; and
a ratio of the peak magnitude of the PPG waveform to a range of spectral magnitudes of the PPG waveform.

**13.** The monitoring device of Claim 8, wherein the monitoring device is configured to be positioned at or within an ear of the subject.

**Patentansprüche**

**1.** Verfahren zum Identifizieren eines besten aus einer Vielzahl von Photoplethysmographie-Kanälen (PPG-Kanälen) eines an einem Probanden angebrachten PPG-Sensors, wobei der PPG-Sensor mindestens einen optischen Detektor (14) und eine Vielzahl von optischen Emittern (12) umfasst, die die Vielzahl von PPG-Kanälen (120) definieren, wobei das Verfahren die folgenden Schritte umfasst:

a) Erfassen von PPG-Daten von dem Probanden unter Verwendung jedes der mehreren PPG-Kanäle über einen Zeitraum; und
b) Verarbeiten der PPG-Daten von jedem PPG-Kanal über einen Prozessor, um eine Vielzahl von PPG-Parametern zu erzeugen;

**gekennzeichnet durch** den weiteren Schritt:
c) Verarbeiten der PPG-Parameter über den Prozessor unter Verwendung eines probabilistischen Modells, um für den Zeitraum einen PPG-Kanal aus der Vielzahl von PPG-Kanälen zu identifizieren, der die geringste Wahrscheinlichkeit aufweist, einen Fehlerwert über einem Schwellenwert für mindestens ein biometrisches Merkmal zu erzeugen, wobei das probabilistische Modell ein maschinelles Lernmodell umfasst, das so konfiguriert ist, dass es die PPG-Parameter als Eingabe empfängt und eine Wahrscheinlichkeit ausgibt, dass ein PPG-Kanal mit einer Fehlerrate verbunden ist, die größer als ein definierter Schwellenwert ist.

2. Verfahren nach Anspruch 1, wobei das Verfahren ferner ein Verarbeiten von Daten aus dem identifizierten besten der mehreren PPG-Kanäle umfasst, um mindestens ein biometrisches Merkmal zu erzeugen;
wobei optional die mindestens eine biometrische Größe ferner mindestens eine der folgenden Größen umfasst: Atemfrequenz des Probanden, Atemvolumen, RR-Intervall (RRi) des Probanden, Blutdruck des Probanden, Blutsauerstoffsättigung des Probanden, Hämodynamik des Probanden, Blutflussvolumen des Probanden und Gewebedurchblutung des Probanden.

3. Verfahren nach Anspruch 1, wobei der PPG-Sensor ferner mindestens einen Bewegungssensor umfasst und wobei das Verfahren ferner umfasst:

Erfassen von Bewegungsdaten über den mindestens einen Bewegungssensor; und
wobei das Verarbeiten der PPG-Daten von jedem PPG-Kanal über den Prozessor zum Erzeugen der Vielzahl von PPG-Parametern das Verarbeiten der Bewegungsdaten von dem mindestens einen Bewegungssensor umfasst.

4. Verfahren nach Anspruch 1, wobei jeder PPG-Parameter PPG-Wellenformdaten oder eine Transformation von PPG-Wellenformdaten umfasst.

5. Verfahren nach Anspruch 1, das ferner den folgenden Schritt umfasst:
d) kontinuierliches Wiederholen der Schritte a) bis c) während einer Überwachungssitzung des Probanden.

6. Verfahren nach Anspruch 5, das ferner umfasst: Bestimmen, ob sich die Person für einen vorbestimmten Zeitraum in Ruhe befunden hat, und als Reaktion auf die Feststellung, dass sich die Person für den vorbestimmten Zeitraum in Ruhe befunden hat, Auswählen eines zuletzt identifizierten PPG-Kanals als den identifizierten PPG-Kanal aus der Vielzahl von PPG-Kanälen und Beenden von Schritt d);
und wobei optional die Überwachungsvorrichtung mindestens einen Bewegungssensor umfasst und wobei das Bestimmen, ob sich die Person für einen vorbestimmten Zeitraum in Ruhe befunden hat, das Verarbeiten von Bewegungsdaten von dem mindestens einen Bewegungssensor und das Überwachen von Bewegungsparametern über den vorbestimmten Zeitraum umfasst.

7. Verfahren nach Anspruch 1, wobei die mehreren PPG-Parameter aus den folgenden ausgewählt werden:

einem Verhältnis der Größe eines pulsierenden Abschnitts einer PPG-Wellenform zu einer Größe eines nicht pulsierenden Abschnitts der PPG-Wellenform;
der nicht-pulsierende Teil der PPG-Wellenform;
ein Verhältnis der Größe des pulsierenden Abschnitts der PPG-Wellenform zu einer Größe schneller Änderungen im pulsierenden Abschnitt der PPG-Wellenform aufgrund von Bewegungsartefakten;
Vertrauen in die Qualität einer PPG-Wellenform;
ein Verhältnis der Größe schneller Veränderungen im nicht pulsierenden Teil der PPG-Wellenform aufgrund von Bewegungsartefakten zur Größe des nicht pulsierenden Teils der PPG-Wellenform;
eine Frequenz, bei der eine Spitzenamplitude der PPG-Wellenform auftritt;
ein Ausmaß eines größten Spektralpeaks der PPG-Wellenform; und
ein Verhältnis der Spitzenamplitude der PPG-Wellenform zu einem Bereich von Spektralamplituden der PPG-Wellenform.

8. Überwachungsvorrichtung, die zum Anbringen an einer Person konfiguriert ist, wobei die Überwachungsvorrichtung umfasst:

einen Photoplethysmographie-Sensor (PPG-Sensor), der so konfiguriert ist, dass er physiologische Informationen von der Person misst, wobei der PPG-Sensor mindestens einen optischen Detektor (14) und eine Vielzahl von optischen Emittern (12) umfasst, die eine Vielzahl von PPG-Kanälen (120) definieren; und
mindestens einen Prozessor, der so konfiguriert ist zum:

Empfangen von PPG-Daten für einen Zeitraum von jedem der mehreren PPG-Kanäle; und
Verarbeiten der PPG-Daten von jedem PPG-Kanal, um eine Vielzahl von PPG-Parametern für den Zeitraum zu erzeugen;

**dadurch gekennzeichnet, dass** der Prozessor ferner konfiguriert ist, um:
die PPG-Parameter unter Verwendung eines probabilistischen Modells zu verarbeiten, um für den Zeitraum einen PPG-Kanal aus der Vielzahl von PPG-Kanälen zu identifizieren, der die geringste Wahrscheinlichkeit aufweist, einen Fehlerwert über einem Schwellenwert für mindestens ein biometrisches Merkmal zu erzeugen, wobei das probabilistische Modell ein maschinelles Lernmodell umfasst, das so konfiguriert ist, dass es die PPG-Parameter als Eingabe empfängt und eine Wahrscheinlichkeit ausgibt, dass ein PPG-Kanal mit einer Fehlerrate verbunden ist, die größer als ein definierter Schwellenwert ist.

9. Überwachungsvorrichtung nach Anspruch 8, wobei der mindestens eine Prozessor ferner so konfiguriert ist, dass er Daten aus dem identifizierten PPG-Kanal aus der Vielzahl von PPG-Kanälen verarbeitet, um mindestens ein biometrisches Merkmal zu erzeugen, wobei das mindestens eine biometrische Merkmal mindestens eines der folgenden Merkmale umfasst: Herzfrequenz des Probanden, Atemfrequenz des Probanden, Atemvolumen des Probanden, RR-Intervall des Probanden, RRi, Blutdruck des Probanden, Blutsauerstoffsättigung des Probanden, Hämodynamik des Probanden, Blutflussvolumen des Probanden und Gewebedurchblutung des Probanden.

10. Überwachungsvorrichtung nach Anspruch 8, die ferner mindestens einen Bewegungssensor umfasst, wobei der mindestens eine Prozessor ferner so konfiguriert ist, dass er Bewegungsdaten aus dem mindestens einen Bewegungssensor mit den PPG-Daten aus jedem PPG-Kanal verarbeitet, um die Vielzahl von PPG-Parametern zu erzeugen.

11. Überwachungsvorrichtung nach Anspruch 8, wobei jeder PPG-Parameter PPG-Wellenformdaten oder eine Transformation von PPG-Wellenformdaten umfasst.

12. Überwachungsvorrichtung nach Anspruch 8, wobei die Vielzahl von PPG-Parametern aus den folgenden ausgewählt ist:

einem Verhältnis der Größe eines pulsierenden Abschnitts einer PPG-Wellenform zur Größe eines nicht pulsierenden Abschnitts der PPG-Wellenform;
der nicht pulsierende Teil der PPG-Wellenform;
ein Verhältnis der Größe des pulsierenden Abschnitts der PPG-Wellenform zu einer Größe schneller Änderungen im pulsierenden Abschnitt der PPG-Wellenform aufgrund von Bewegungsartefakten;
Vertrauen in die Qualität einer PPG-Wellenform;
ein Verhältnis der Größe schneller Veränderungen im nicht pulsierenden Teil der PPG-Wellenform aufgrund von Bewegungsartefakten zur Größe des nicht pulsierenden Teils der PPG-Wellenform;
eine Frequenz, bei der eine Spitzenamplitude der PPG-Wellenform auftritt;
einem Ausmaß eines größten Spektralpeaks der PPG-Wellenform; und
ein Verhältnis der Spitzenamplitude der PPG-Wellenform zu einem Bereich von Spektralamplituden der PPG-Wellenform.

13. Überwachungsvorrichtung nach Anspruch 8, wobei die Überwachungsvorrichtung so konfiguriert ist, dass sie an oder in einem Ohr des Probanden positioniert werden kann.

**Revendications**

1. Un procédé d'identification d'un meilleur canal parmi une pluralité de canaux de photopléthysmographie, PPG, d'un capteur PPG fixé à un individu, le capteur PPG comprenant au moins un détecteur optique (14) et une pluralité d'émetteurs optiques (12) qui définissent la pluralité de canaux PPG (120), le procédé comprenant les étapes suivantes :

a) la détection de données PPG en provenance de l'individu en utilisant chacun de la pluralité de canaux PPG pendant une période de temps ; et
b) le traitement des données PPG provenant de chaque canal PPG, via un processeur, pour générer une pluralité de paramètres PPG ;

**caractérisé par**, en outre, l'étape suivante :
c) le traitement des paramètres PPG, via le processeur en utilisant un modèle probabiliste, pour identifier pendant la période de temps un canal PPG de la pluralité de canaux PPG qui présente une probabilité la plus faible de générer une valeur d'erreur audessus d'un seuil pour au moins une biométrie, le modèle probabiliste comprenant un modèle d'apprentissage machine configuré pour recevoir en entrée les paramètres PPG et délivrer en sortie une probabilité qu'un canal PPG soit associé à un taux d'erreurs supérieur à un seuil défini.

**2.** Le procédé de la revendication 1, dans lequel le procédé comprend en outre le traitement de données provenant du meilleur canal identifié de la pluralité de canaux PPG pour générer au moins une biométrie ;

éventuellement dans lequel l'au moins une biométrie comprend en outre au moins l'un d'entre un rythme respiratoire de l'individu, un volume respiratoire, un intervalle RR (RRi) de l'individu, une pression sanguine de l'individu, un taux d'oxygène sanguin de l'individu, une hémodynamique de l'individu, un volume de flux sanguin de l'individu, et une perfusion tissulaire de l'individu.

**3.** Le procédé de la revendication 1, dans lequel le capteur PPG comprend en outre au moins un capteur de mouvement, et dans lequel le procédé comprend en outre :

la détection de données de mouvement via l'au moins un capteur de mouvement ; et
dans lequel le traitement, via le processeur, des données PPG en provenance de chaque canal PPG pour générer la pluralité de paramètres PPG comprend le traitement des données de mouvement provenant de l'au moins un capteur de mouvement.

**4.** Le procédé de la revendication 1, dans lequel chaque paramètre PPG comprend des données de forme d'onde PPG ou une transformée de données de forme d'onde PPG.

**5.** Le procédé de la revendication 1, comprenant en outre l'étape suivante :
d) la répétition continuelle des étapes a) à c) pendant une session de suivi de l'individu.

**6.** Le procédé de la revendication 5, comprenant en outre la détermination si l'individu a été au repos pendant une période de temps prédéterminée, et, en réponse à la détermination que l'individu a été au repos pendant la période de temps prédéterminée, la sélection d'un dernier canal PPG identifié comme étant le canal PPG identifié de la pluralité de canaux PPG et l'achèvement de l'étape d) ;
et éventuellement dans lequel le dispositif de suivi comprend au moins un capteur de mouvement, et dans lequel la détermination si l'individu a été au repos pendant une période de temps prédéterminée comprend le traitement de données de mouvement provenant de l'au moins un capteur de mouvement et le suivi de paramètres de mouvement sur la période de temps prédéterminée.

**7.** Le procédé de la revendication 1, dans lequel la pluralité de paramètres PPG sont choisis parmi les suivants :

un ratio d'un niveau d'une partie pulsatile d'une forme d'onde PPG par rapport à un niveau d'une partie non pulsatile de la forme d'onde PPG ;
la partie non pulsatile de la forme d'onde PPG ;
un ratio du niveau de la partie pulsatile de la forme d'onde PPG par rapport à un niveau de changements rapides de la partie pulsatile de la forme d'onde PPG qui résultent d'artefacts de mouvement ;
une confiance dans la qualité d'une forme d'onde PPG ;
un ratio d'un niveau de changements rapides dans la partie non pulsatile de la forme d'onde PPG qui résultent d'artefacts de mouvement par rapport au niveau de la partie non pulsatile de la forme d'onde PPG ;
une fréquence à laquelle apparait un niveau de pic de la forme d'onde PPG ;
un niveau d'un plus grand pic spectral de la forme d'onde PPG ; et
un ratio du niveau de pic de la forme d'onde PPG par rapport à une plage de niveaux spectraux de la forme d'onde PPG.

**8.** Un dispositif de suivi configuré pour être fixé à un individu, le dispositif de suivi comprenant :

un capteur photopléthysmographique, PPG, configuré pour mesurer des informations physiologiques provenant de l'individu, le capteur PPG comprenant au moins un détecteur optique (14) et une pluralité d'émetteurs optiques (12) qui définissent une pluralité de canaux PPG (120) ; et
au moins un processeur configuré pour :

obtenir des données PPG pendant une période de temps en provenance de chacun de la pluralité de canaux PPG ; et
traiter les données PPG provenant de chaque canal PPG pour générer une pluralité de paramètres PPG pendant la période de temps ;
**caractérisé en ce que** le processeur est en outre configuré pour :
traiter les paramètres PPG en utilisant un modèle probabiliste pour identifier pendant la période de temps un canal PPG de la pluralité de canaux PPG qui présente une probabilité la plus faible de générer une valeur d'erreur au-dessus d'un seuil pour au moins une biométrie, le modèle probabiliste comprenant un modèle d'apprentissage machine configuré pour recevoir en entrée les paramètres PPG et délivrer en sortie une probabilité qu'un canal PPG soit associé à un taux d'erreurs supérieur à un seuil défini.

**9.** Le dispositif de suivi de la revendication 8, dans lequel l'au moins un processeur est en outre configuré pour traiter des données provenant du canal PPG identifié de la pluralité de canaux PPG pour générer au moins une biométrie, dans lequel l'au moins une biométrie comprend en outre au moins l'un d'entre un rythme respiratoire de l'individu, un volume respiratoire de l'individu, un intervalle RR (RRi) de l'individu, une pression sanguine de l'individu, un taux d'oxygène sanguin de l'individu, une hémodynamique de l'individu, un volume de flux sanguin de l'individu, et une perfusion tissulaire de l'individu.

**10.** Le dispositif de suivi de la revendication 8, comprenant en outre au moins un capteur de mouvement, et dans lequel l'au moins un processeur est en outre configuré pour traiter les données de mouvement provenant de l'au moins un capteur de mouvement avec les données PPG provenant de chaque canal PPG pour générer la pluralité de paramètres PPG.

**11.** Le dispositif de suivi de la revendication 8, dans lequel chaque paramètre PPG comprend des données de forme d'onde PPG ou une transformée de données de forme d'onde PPG.

**12.** Le dispositif de suivi de la revendication 8, dans lequel la pluralité de paramètres PPG sont choisis parmi les suivants :

un ratio d'un niveau d'une partie pulsatile d'une forme d'onde PPG par rapport à un niveau d'une partie non pulsatile de la forme d'onde PPG ;
la partie non pulsatile de la forme d'onde PPG ;
un ratio du niveau de la partie pulsatile de la forme d'onde PPG par rapport à un niveau de changements rapides de la partie pulsatile de la forme d'onde PPG qui résultent d'artefacts de mouvement ;
une confiance dans la qualité d'une forme d'onde PPG ;
un ratio d'un niveau de changements rapides dans la partie non pulsatile de la forme d'onde PPG qui résultent d'artefacts de mouvement par rapport au niveau de la partie non pulsatile de la forme d'onde PPG ;
une fréquence à laquelle apparait un niveau de pic de la forme d'onde PPG ;
un niveau d'un plus grand pic spectral de la forme d'onde PPG ; et
un ratio du niveau de pic de la forme d'onde PPG par rapport à une plage de niveaux spectraux de la forme d'onde PPG.

**13.** Le dispositif de suivi de la revendication 8, dans lequel le dispositif de suivi est configuré pour être positionné au niveau, ou à l'intérieur, d'une oreille de

10

Bottom View

14  12

12

12

12

20
PPG HR
Sensor

20  Side View

FIG. 1A

Optical detector *14*

Array of individual
optical emitters (LEDs) *12*

Fig. 1B

FIG. 2

EP 3 941 344 B1

Connector
Cable

200

D1

D2

Audio
Channel

U1

U2

FIG. 3

Parameters Used in Hunting-Mode for Accurate Heart Rate Monitoring
in RIC Earpiece & Wrist Example

| Parameter | Description |
|---|---|
| AC/DC ratio | The magnitude of the pulsatile PPG signal (due to heartbeats) compared to the magnitude of the non-pulsatile PPG signal |
| Non-pulsatile signal | The "DC" or non-pulsatile portion of the PPG waveform |
| AC/Fidget | The magnitude of the pulsatile PPG signal compared to the magnitude of rapid changes in PPG signal due to noise-inducing motion artifacts |
| Confidence | Confidence in the quality of the PPG signal |
| Fidget/DC | The magnitude of rapid changes in the PPG signal due to noise-inducing motion artifacts compared to the magnitude of the non-pulsatile PPG signal |
| PPG peak frequency | The frequency at which the PPG peak magnitude occurs. |

FIG. 4

300

Input
- PPG Parameters
- Motion Sensor Parameters

Neural Net

Output
- Best PPG Channel

FIG. 5

Results of training with all participants except 1 (leaving one to test)

Compared against choosing the channel with the best mape for each test

| Swiss3, SRT4, Left, BE, Back6 (U1) | | | | Swiss3, SRT4, Left, BE, Front6 (U2) | | | | Swiss3, SRT4, Left, RIC, BACK6 (U1) | | | | Swiss3, SRT4, Left, RIC, Front6 (U2) | | | |
|---|---|---|---|---|---|---|---|---|---|---|---|---|---|---|---|
| Participant | MAPE Using "Best Of" Algorithm | MAPE of Best Channel | Better or Worse? | Participant | MAPE Using "Best Of" Algorithm | MAPE of Best Channel | Better or Worse? | Participant | MAPE Using "Best Of" Algorithm | MAPE of Best Channel | Better or Worse? | Participant | MAPE Using "Best Of" Algorithm | MAPE of Best Channel | Better or Worse? |
| ShSt | 3.20% | 3.88% | **Better** | ShSt | 3.91% | 4.30% | **Better** | ShSt | 4.69% | 5.44% | **Better** | ShSt | 5.95% | 8.83% | **Better** |

| Swiss3, SRT4, Right, BE, Back6 (U1) | | | | Swiss3, SRT4, Right, BE, Front6 (U2) | | | | Swiss3, SRT4, Right, RIC, Back6 (U1) | | | | Swiss3, SRT4, Right, RIC, Front6 (U2) | | | |
|---|---|---|---|---|---|---|---|---|---|---|---|---|---|---|---|
| Participant | MAPE Using "Best Of" Algorithm | MAPE of Best Channel | Better or Worse? | Participant | MAPE Using "Best Of" Algorithm | MAPE of Best Channel | Better or Worse? | Participant | MAPE Using "Best Of" Algorithm | MAPE of Best Channel | Better or Worse? | Participant | MAPE Using "Best Of" Algorithm | MAPE of Best Channel | Better or Worse? |
| ShSt | 2.18% | 3.21% | **Better** | ShSt | 2.84% | 3.87% | **Better** | ShSt | 3.72% | 4.45% | **Better** | ShSt | 4.65% | 4.49% | **Better** |

FIG. 6

EP 3 941 344 B1

EP 3 941 344 B1

```
500 ──┐  ┌─────────────────────────┐
       └─►│   Dynamic hunting mode   │◄─────────────────┐
          └─────────────────────────┘                   │
                     │                                   │
          502        ▼                                   │
            ┌─────────────────┐                          │
            ╱                   ╲          No             │
           ╱   Best PPG channel  ╲─────────────────────►│
           ╲   identified?       ╱                       │
            ╲                   ╱                         │
             └─────────────────┘                         │
                     │ Yes          504                  │
                     ▼                                    │
          ┌──────────────────────────────┐               │
          │   Generate biometrics via     │              │
          │   dynamically best PPG channel;│◄────────────┤
          │   Continue with dynamic        │             │
          │   hunting-mode                 │             │
          └──────────────────────────────┘              │
                     │          506                      │
                     ▼                                    │
            ┌─────────────────┐                          │
            ╱                   ╲          No             │
     Yes   ╱   Subject at       ╲────────────────────────┘
   ┌──────╲   rest (for a       ╱
   │       ╲  sufficient       ╱
   │        ╲ period of time)? ╱
   │         └─────────────────┘
   │  508
   ▼
┌──────────────────────────────────────┐
│   Hunting-mode turned off;            │
│   Generate biometrics via last best   │◄─┐
│   PPG channel;                        │  │
│   Other PPG channels turned off       │  │
└──────────────────────────────────────┘  │
                     │          510        │
                     ▼                      │
            ┌─────────────────┐             │
     Yes   ╱                   ╲   No        │
   ┌──────╱   Signal quality    ╲───────────┘
   │      ╲  at or above desired ╱
   │       ╲ threshold?         ╱
   │        └─────────────────┘
   └───────────────────────────
```

FIG. 7

Parameters Used in Hunting-Mode for Accurate Heart Rate Monitoring
in RIC Earpiece & Wrist Example

| Parameter | Description |
|---|---|
| Peak Magnitude | The magnitude of the largest spectral peak of the PPG signal (the spectral magnitude at the PPG Peak Frequency for a multi-second segment of PPG data) |
| Signal Quality | The ratio of PPG Peak Magnitude to the range of PPG spectral magnitudes (a comparison of the PPG Peak Magnitude to the range of spectral magnitudes in a multi-second segment of PPG data) |

FIG. 8

FIG. 9

EP 3 941 344 B1

Exemplary spectrogram of PPG signal without active motion noise removal

Fig. 10A

Exemplary spectrogram of PPG signal
following active motion noise removal

$\omega_{HR}$ = Peak Frequency

$A(\omega_{HR})$ = Peak Magnitude

$A(\omega)$

$\omega$

Fig. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62880442
- US 8251903 B
- US 2018353075 A, Boston Scientific
- EP 1297784 A, CSEM
- US 2017071487 A, Whoop, Inc.

- US 10015582 B
- US 62733327
- US 10631740 B
- US 8888701 B
- US 9794653 B